# EUROPEAN PATENT SPECIFICATION

(11) **EP 2 375 912 B1**
(45) Date of publication and mention of the grant of the patent: **23.08.2017**
(21) Application number: 10700636.3
(22) Date of filing: 12.01.2010
(51) Int. Cl.: A23G 3/36, A23L 2/56, A23L 27/20, A61K 31/16, A61P 11/04

(54) **COMPOSITIONS AND METHODS FOR THE TREATMENT OF THROAT DISCOMFORT**
ZUSAMMENSETZUNGEN UND VERFAHREN ZUR BEHANDLUNG VON HALSSCHMERZEN
COMPOSITIONS ET PROCÉDÉS POUR LE TRAITEMENT D'UN INCONFORT DE LA GORGE

(30) Priority: 15.01.2009 US 144846 P
(43) Date of publication of application: 19.10.2011
(73) Proprietor: Intercontinental Great Brands LLC, East Hanover, NJ 07936 (US)
(72) Inventor: CORNEC, Michel, Morristown, NJ 07960 (US); CORREA, Beth, Ann, Morristown, NJ 07960 (US); HARNISH, Michelle, Sparta, NJ 07871 (US); HARVEY, Joan, E., Easton, PA 18045 (US); LYNCH, Caroline, Hoboken, NJ 07030 (US); SCHWARTZ, Danielle, Hamburg, NJ 07419 (US); WATSON, Deborah, L., Haskell, NJ 07420 (US)
(74) Representative: Wilson Gunn
(86) International application number: PCT/US2010/020685
(87) International publication number: WO 2010/083133

(56) References cited:
- WO-A1-2008/039522
- WO-A1-2008/124667
- GB-A- 1 421 743
- GB-A- 1 421 744

## Description

### BACKGROUND OF THE INVENTION

Throat discomfort, which includes throat pain and throat irritation, is a common symptom of viral and bacterial infections, allergies, breathing polluted air, and smoking. Throat lozenges constitute one category of over-the-counter treatments for throat discomfort. The most common active ingredient in such lozenges is menthol, which is sometimes used in combination with eucalyptus oil. Menthol exerts a physiological cooling effect that can soothe a sore throat. However, the use of menthol in throat lozenges suffers from two significant drawbacks. First, concentrations of menthol that are effective for throat soothing are also accompanied by a bitterness that many people find objectionable. Second, the physiological cooling effect of a menthol-containing lozenge is felt primarily in the mouth rather than the throat. There is therefore a desire for throat lozenge compositions that exhibit reduced bitterness and increased selectivity for cooling in the throat rather than the mouth.

### BRIEF DESCRIPTION OF THE INVENTION

Provided in the invention is a confection, comprising: at least 80 weight percent of a confectionery base; about 0.005 to about 0.5 weight percent of monomenthyl glutarate, and about 0.03 to about 0.4 weight percent of N-ethyl-2,2-diisopropylbutanamide; wherein all weight percents are based on the total weight of the confection.

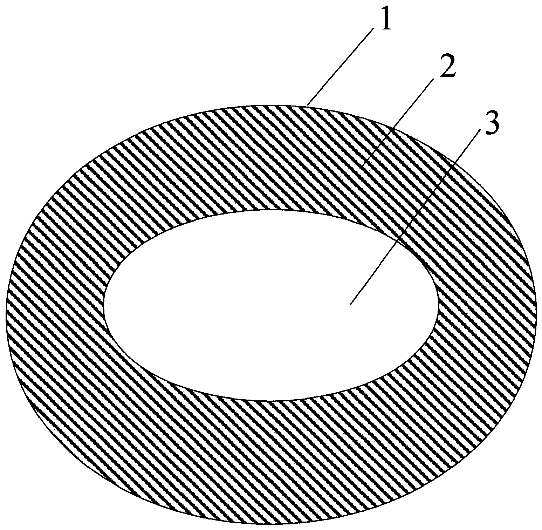

### BRIEF DESCRIPTION OF THE FIGURE

The FIGURE is a side, cross-sectional view of a center-filled confection.

### DETAILED DESCRIPTION OF THE INVENTION

The present inventors have observed that N-ethyl-2,2-diisopropylbutanamide is a particularly effective for throat soothing. At therapeutically effective doses it is much less bitter than menthol. Moreover, it exhibits selectivity for throat cooling over mouth cooling. This selectivity for throat cooling is particularly surprising given that a very close chemical relative, N,2,3-trimethyl-2-isopropylbutanamide, exerts its cooling effect primarily in the mouth rather than the throat. The structures of N-ethyl-2,2-diisopropylbutanamide and N,2,3-trimethyl-2-isopropylbutanamide, which differ only by two methylene (-CH₂-) groups, are shown below.

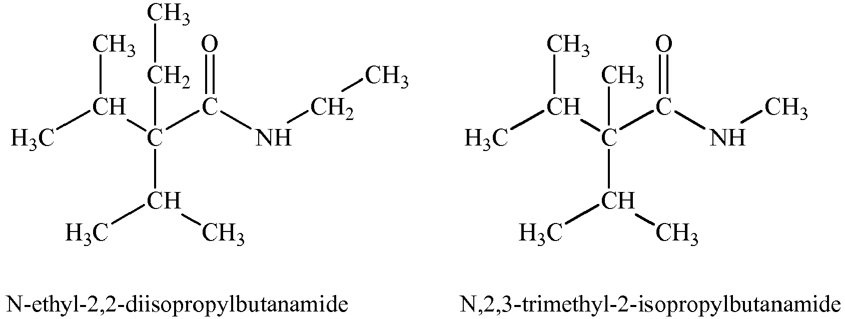

A method of treating throat discomfort, comprising administering (e.g., via mouth) to a subject in need thereof an effective amount of N-ethyl-2,2-diisopropylbutanamide is described. N-Ethyl-2,2-diisopropylbutanamide is a known compound, having been described in Great Britain Patent No. 1,421,744 to Rowsell et al. In that reference, it is one of a family of acyclic carboxamides that is characterized as exhibiting physiological cooling activity. In particular, the family of compounds is characterized as "having a physiological cooling effect on the skin and on the mucous membranes of the body, particularly the mucous membranes of the nose and bronchial tract." GB 1,421,744 to Rowsell et al., page 1, lines 12-15. The Rowsell patent includes a Table in which each compound's "cooling activity" is indicated by a scale of one to five asterisks, but the procedure by which cooling activity was evaluated is not provided, and there is no specific indication of throat-cooling activity.

For the treatment of throat discomfort, N-ethyl-2,2-diisopropylbutanamide can be administered by mouth in a variety of confectionery forms, including hard candies, soft candies, center-filled confections, jellies, and films.

The effective amount of N-ethyl-2,2-diisopropylbutanamide may be administered in the form of a confection having a mass of about 2 to about 10 grams and comprising about 0.03 to about 0.4 weight percent ofN-ethyl-2,2-diisopropylbutanamide, based on the weight of the confection. Within the range of about 2 to about 10 grams, the confection weight can be about 3 to about 8 grams, specifically about 4 to about 6 grams. Within the range of about 0.03 to about 0.4 weight percent, the concentration of N-ethyl-2,2-diisopropylbutanamide can be about 0.05 to about 0.25 weight percent, specifically about 0.06 to about 0.12 weight percent.

In addition to the N-ethyl-2,2-diisopropylbutanamide, the confection comprises a confectionery base. As used herein, the term "confectionery base" includes any ingredient or group of ingredients that represent the bulk of the confectionery composition and provide the confectionery composition with its structural integrity and to which other ingredients are added. Examples of confectionery bases include sucrose, dextrose, maltose, dextrin, xylose, ribose, glucose, mannose, galactose, fructose, lactose, invert sugar, fructooligosaccharide syrups, partially hydrolyzed starch, corn syrup solids (e.g., in the form of high fructose corn syrup), sorbitol, mannitol, maltitol, xylitol, erythritol, polysaccharide polyols, maltitol syrups, hydrogenated starch hydrolysates, polydextrose, and combinations thereof. Still other examples can incorporate fats and hydrocolloids as in bases that include thin boiling starch mixed with sucrose, corn syrup and hydrogenated vegetable oil or bases that include pectin, sucrose, and corn syrup. The term "confectionery base" does not include the gum bases used in the formulation of chewing gums.

In the confection, the concentration of confectionery base can be at least 80 weight percent, specifically at least 85 weight percent, more specifically at least 90 weight percent, still more specifically at least 95 weight percent, of the total weight of the confection.

In order to emphasize throat cooling over mouth cooling, N-ethyl-2,2-diisopropylbutanamide can be used as the sole or primary physiological cooling agent. Alternatively, e.g., when a combination of mouth cooling and throat cooling is desired, N-ethyl-2,2-diisopropylbutanamide can be used in combination with other physiological cooling agents. In general, it should be noted that that the use of cooling agents in gum and confections pose different formulation challenges. In gum, release of the cooling agents is influenced by partitioning of the cooling agents between the gum base and the essentially aqueous environment of the mouth. In contrast, release of cooling agents from confections is controlled largely by the surface area and dissolution rate of the hard candy. Therefore, strategies to achieve sustained cooling sensation in gum by using multiple cooling agents with different water solubilities are unlikely to achieve the same effect in a confection.

In the present confections, a desirable balance between mouth cooling and throat cooling can be attained when the confection comprises, based on the total weight of the confection, about 0.03 to about 0.4 weight percent N-ethyl-2,2-diisopropylbutanamide and about 0.005 to about 0.5 weight percent of monomenthyl glutarate. Within the range of about 0.005 to about 0.5 weight percent, the amount of monomenthyl glutarate can be about 0.02 to about 0.3 weight percent, specifically about 0.04 to about 0.2 weight percent, more specifically about 0.08 to about 0.16 weight percent.

Combinations ofN-ethyl-2,2-diisopropylbutanamide with other physiological cooling agents are possible. Besides monomenthyl glutarate and N-ethyl-p-menthane-3-carboxamide, other physiological cooling agents that can be combined with N-ethyl-2,2-diisopropylbutanamide include N-(2-hydroxyethyl)-2-isopropyl-2,3-dimethylbutanamide, N-(3-ethoxypropyl)-2-isopropyl-2,3-dimethylbutanamide, N-(3-propoxypropyl)-2-isopropyl-2,3-dimethylbutanamide, N-(3-butoxypropyl)-2-isopropyl-2,3-dimethylbutanamide, N-ethyl-p-menthane-3-carboxamide (WS-3), the ethyl ester of N-[[5-methyl-2-(1-methylethyl)cyclohexyl]carbonyl]glycine (ethyl 3-(p-menthane-3-carboxamido)acetate; WS-5), N-(1,1-dimethyl-2-hydroxyethyl)-2,2-diethylbutanamide, isopulegol, 3-(L-menthoxy)propane-1,2-diol, 3-(L-menthoxy)-2-methylpropane-1,2-diol, menthane diols such as p-menthane-2,3-diol and p-menthane-3,8-diol, 6-isopropyl-9-methyl-1,4-dioxaspiro[4,5]decane-2-methanol, menthyl succinate and its alkaline earth metal salts, trimethylcyclohexanol, N-ethyl-2-isopropyl-5-methylcyclohexanecarboxamide, Japanese mint oil, peppermint oil, menthone, isomenthone, menthone glycerol ketals, menthyl lactate, 3-(L-menthoxy)ethan-1-ol, 3-(L-menthoxy)propan-1-ol, 3-(L-menthoxy)butan-1-ol, L-menthylacetic acid N-ethylamide, L-menthyl-4-hydroxypentanoate, L-menthyl-3-hydroxybutyrate, N,2,3-trimethyl-2-(1-methylethyl)-butanamide, N-ethyl-trans-2-cis-6-nonadienamide, N,N-dimethyl menthyl succinamide, menthyl pyrrolidone carboxylate, xylitol, erythritol, menthane, menthone ketals, substituted p-menthanes, acyclic carboxamides, substituted cyclohexanamides, substituted cyclohexane carboxamides, substituted menthanols, hydroxymethyl derivatives of p-menthane, 2-mercapto-cyclodecanone, 2-isopropyl-5-methylcyclohexanol, cyclohexanamides, menthyl acetate, menthyl salicylate, N,2,3-trimethyl-2-isopropylbutanamide (WS-23), icilin, camphor, borneol, eucalyptus oil, peppermint oil, bornyl acetate, lavender oil, wasabi extracts, horseradish extracts, 3,1-menthoxypropane 1,2-diol, and the like, and combinations thereof. These and other suitable cooling agents are further described in, for example, U.S. Patent Nos. 4,032,661 and 4,230,688 of Rowsell et al., 4,459,425 to Amano et al., 4,136,163 to Watson et al., 5,266,592 to Grub et al., 6,627,233 to Wolf et al., and 7,030,273 to Sun.

The confection can, optionally, further comprise menthol in an amount of about 0.01 to about 0.2 weight percent, specifically about 0.02 to about 0.15 weight percent, more specifically about 0.05 to about 0.13 weight percent, based on the total weight of the confection.

In some embodiments, the confection further comprises eucalyptus oil in an amount of about 0.001 to about 0.02 weight percent, specifically about 0.002 to about 0.015 weight percent, more specifically about 0.003 to about 0.01 weight percent, based on the total weight of the confection.

A confection, comprising: at least 80 weight percent of a confectionery base; about 0.005 to about 0.5 weight percent of monomenthyl glutarate, and about 0.03 to about 0.4 weight percent of N-ethyl-2,2-diisopropylbutanamide; wherein all weight percents are based on the total weight of the confection is provided as part of the invention. Within the limitation of at least 80 weight percent, the amount of confectionery based can be at least 85 weight percent, specifically at least 90 weight percent, more specifically at least 95 weight percent. Within the range of about 0.005 to about 0.5 weight percent, the amount of monomenthyl glutarate can be about 0.02 to about 0.3 weight percent, specifically about 0.04 to about 0.2 weight percent, more specifically about 0.08 to about 0.16 weight percent. Within the range of about 0.03 to about 0.4 weight percent, the amount of N-ethyl-2,2-diisopropylbutanamide can be about 0.05 to about 0.25, specifically about 0.06 to about 0.12 weight percent. The confection can be hard candy confection, a soft candy confection, a center-filled confection, a jelly, or a film. As used herein, the term "hard candy" is synonymous with "hard boiled candy" and "high boiled candy," and includes all confectionery compositions wherein the saccharide component(s) are heated to temperatures high enough to remove most of their moisture or where saccharide component(s) are blended without cooking such that the finished product water content is less than or equal to five weight percent.

The confection can, optionally, further comprise menthol and/or eucalyptus oil. When present, the amount of menthol is about 0.01 to about 0.2 weight percent, specifically about 0.02 to about 0.15 weight percent, more specifically about 0.05 to about 0.13 weight percent. When present, the amount of eucalyptus oil is about 0.001 to about 0.02 weight percent, specifically about 0.002 to about 0.015 weight percent, specifically about 0.003 to about 0.01 weight percent.

The confection can be a solid mass of uniform composition. Alternatively, the confection can comprise a center filling that is softer than the candy shell that surrounds it. For example the confection can be a center-filled confection comprising a shell and a filling partially or fully surrounded by the shell. A cross sectional view of a representative center-filled confection is presented in the Figure, where the center-filled confection 1 comprises a shell 2 surrounding a center filling 3. In some embodiments, the confection comprises about 60 to about 95 weight percent, specifically about 70 to about 92 weight percent, of the shell, and about 5 to about 40 weight percent, specifically about 8 to about 30 weight percent, of the center filling. The N-ethyl-2,2-diisopropylbutanamide can be included in the shell, the center filling, or both.

Some embodiments are directed to center-filled products, such as center-filled candy. Such products generally include a center-fill region and a region that at least partially surrounds the center-fill region. The region that at least partially surrounds the center-fill region can be a hard candy or soft candy composition. Suitable candy, or confectionery, compositions are described above.

The center-fill region in some embodiments can be a liquid, solid or semi-solid, gas, or the like. For example, in some embodiments, the center-fill region can be a powdered confectionery composition. Center-fill compositions can include any of the sweeteners, flavors, cooling agents, coloring agents and the like described above.

In some embodiments, the center-fill region can be substantially or completely filled with the liquid, solid, semi-solid or gaseous center-fill composition. In some other embodiments, the center-fill region can be only partially filled with the liquid, solid, semi-solid or gaseous center-fill composition.

In some embodiments, the center-fill region can include two or more center-fill compositions. The two or more center-fill compositions can be the same or different forms. For example, some embodiments can contain a mixture of two or more distinct liquids. Similarly, some embodiments can contain two or more distinct solids, semi-solids, or gases in the center-fill region. Mixtures of different center-fill forms also can be included in some embodiments. For example, a liquid and a solid can be included in the center-fill region. The two or more liquids, solids, semi-solids and/or gases employed in the center-fill region can be included in the same or different amounts and can have similar or distinct characteristics. More specifically, in some embodiments, the two or more center-fill compositions can differ in a variety of characteristics, such as, viscosity, color, flavor, taste, texture, sensation, ingredient components, functional components, sweeteners, or the like.

In some embodiments, the center-fill composition can include a viscosity modifier. Suitable viscosity modifiers include, for example, glycerin, lecithin, medium chain triglycerides, and mixtures thereof.

In some embodiments, the center-fill composition also can include non-liquid components, such as, for example, flavor beads, fruit particles, nut particles, flavor particles, gelatin beads or portions, and the like.

In some embodiments, the sensate composition including N-ethyl-2,2-diisopropylbutanamide can be present in the center-fill region, the shell region, which at least partially surrounds the center-fill region, or both regions. Some embodiments can include a first cooling composition in the center-fill region and a second cooling composition in the candy or gum region. The second cooling composition can be the same as or different from the first.

Some center-fill embodiments optionally can include a third, or coating, region. In some embodiments, the coating also can be referred to as the "outermost region" of the product. The coating can at least partially surround the shell region. The coating can be any conventional sugar or sugarless coating, which forms an exterior surface on the center-filled product.

A variety of coating processes are known. In some embodiments, a hard coating is applied in numerous thin layers of material in order to form an appropriate uniform coated and finished quality surface on the products. The coating material, which can include sugar, maltitol, sorbitol, or any other polyol, including those described herein, and optionally flavoring, is sprayed onto the candy pieces as they pass through a coating mechanism or a coating tunnel and are tumbled and rotated therein. In addition, conditioned air is circulated or forced into the coating tunnel or mechanism in order to dry each of the successive coating layers on the formed products. In some embodiments, the coating, or outermost region, can be formed by lamination, dual or multiple extrusion, or any other process that creates an outermost region.

In addition to hard coatings, other types of coatings can include gumming or glazing, soft coating, smoothing, frosting, sanding, and wet crystallization. These coating processes are described in more detail in U.S. Patent No. 5,527,542 to Serpelloni et al. Further, the exterior or outermost region can be a lipid material such as an oil. This lipid material can be applied to the product by any suitable means.

The center-fill can be a powder center filling. For example, in some embodiments, the shell comprises a hard candy shell composition comprising, based on the total weight of the confection composition, at least 80 weight percent of a confectionery base; about 0.005 to about 0.5 weight percent of monomenthyl glutarate, and about 0.03 to about 0.4 weight percent of N-ethyl-2,2-diisopropylbutanamide; and the center filling comprises a powder center filling composition comprising, based on the total weight of the fluid center filling composition, about 90 to about 99.99 weight percent of a powdered confectionery base; about 0.5 to about 25 weight percent of a powder flow agent selected from the group consisting of powdered cellulose, magnesium stearate, stearic acid, paraffin and microcrystalline waxes, polyethylene waxes, mineral and other lubricating oils, talc, silicone dioxide, lactose, calcium citrate, and mixtures thereof; about 0.005 to about 0.5 weight percent of monomenthyl glutarate, and about 0.03 to about 0.4 weight percent of N-ethyl-2,2-diisopropylbutanamide. In some embodiments, the hard candy composition, the fluid center filling composition, or both comprises monomenthyl glutarate.

In some embodiments, the center-fill is a fluid center filling that is softer than the shell, and it can be soft enough to flow at room temperature. For example, in some embodiments, the fluid center filling has a viscosity of about 10 to about 200,000 centipoise, specifically about 100 to about 50,000 centipoise, at 25°C. The fluid center filling composition typically has a water content of about 7 to 13 weight percent, based on the weight of the fluid center filling composition. The fluid center filling composition also can have about 5 to about 20 weight percent of a viscosity modifier, such as glycerin, lecithin, medium chain triglycerides, and mixtures thereof.

In some embodiments of the center-filled confection, the shell comprises a hard candy shell composition comprising, based on the total weight of the confection composition, at least 80 weight percent (specifically at least 85 weight percent, more specifically at least 90 weight percent, still more specifically at least 95 weight percent) of a confectionery base; about 0.005 to about 0.5 weight percent (specifically about 0.02 to about 0.3 weight percent, more specifically about 0.04 to about 0.2 weight percent, still more specifically about 0.08 to about 0.16 weight percent) of monomenthyl glutarate, and about 0.03 to about 0.4 weight percent (specifically about 0.05 to about 0.25 weight percent, more specifically about 0.06 to about 0.12 weight percent) ofN-ethyl-2,2-diisopropylbutanamide; and the center filling comprises a fluid center filling composition comprising, based on the total weight of the fluid center filling composition, about 70 to about 95 weight percent of a confectionery base; about 5 to about 20 weight percent of a viscosity modifier selected from the group consisting of glycerin, lecithin, medium chain triglycerides, and mixtures thereof; about 0.005 to about 0.5 weight percent (specifically about 0.02 to about 0.3 weight percent, more specifically about 0.04 to about 0.2 weight percent, even more specifically about 0.08 to about 0.16 weight percent) of monomenthyl glutarate, and about 0.03 to about 0.4 weight percent (specifically about 0.05 to about 0.25 weight percent, more specifically about 0.06 to about 0.12 weight percent) of N-ethyl-2,2-diisopropylbutanamide.

In some embodiments, the hard candy composition, the fluid center filling composition, or both comprises monomenthyl glutarate.

In a very specific embodiment of the center-filled confection, it comprises, based on the total weight of the center-filled confection, about 70 to about 95 weight percent of the hard candy shell and about 5 to about 30 weight percent of the fluid center filling; the hard candy shell composition comprises, based on the total weight of the hard candy shell composition, about 90 to about 99.5 weight percent of the confectionery base, about 0.08 to about 0.16 weight percent monomenthyl glutarate, about 0.06 to about 0.12 weight percent N-ethyl-2,2-diisopropylbutanamide, and about 0.05 to about 0.13 weight percent menthol; the fluid center filling comprises a fluid center filling composition comprising, based on the total weight of the fluid center filling, about 75 to about 90 weight percent confectionery base, about 10 to about 17 weight percent glycerin, about 0.08 to about 0.16 weight percent monomenthyl glutarate, about 0.06 to about 0.12 weight percent N-ethyl-2,2-diisopropylbutanamide, and about 0.02 to about 0.07 weight percent menthol.

The confection can, optionally, further include a taste potentiator. Taste potentiators are substances capable of reducing or eliminating undesirable tastes in edible substances. Taste potentiators can also serve to enhance desirable tastes in edible substances such as sweetness potentiators that increase sweetness intensity. In the context of cooling agents, taste potentiators can be effective to reduce or eliminate bitterness, undesired mintiness, or other undesired taste. The taste potentiator compositions can have controlled-release properties. The taste potentiator can work synergistically with the cooling agent to enhance the perception of the cooling agent. In some embodiments, delivery of a sweetener in combination with a taste potentiator can enhance the sweet taste upon consumption of the composition. The incorporation of the potentiator, therefore, can allow for reduced amounts of cooling agent and/or sweetener without compromising the levels of cooling and sweetness provided by the composition.

Any of a variety of substances that function as taste potentiators can be employed. For instance, suitable taste potentiators include water-soluble taste potentiators, such as neohesperidin dihydrochalcone, chlorogenic acid, alapyridaine, cynarin, miraculin, glupyridaine, pyridinium-betain compounds, glutamates, such as monosodium glutamate and monopotassium glutamate, neotame, thaumatin, tagatose, trehalose, salts, such as sodium chloride, monoammonium glycyrrhizinate, vanilla extract (in ethyl alcohol), water-soluble sugar acids, potassium chloride, sodium acid sulfate, water-soluble hydrolyzed vegetable proteins, water-soluble hydrolyzed animal proteins, water-soluble yeast extracts, adenosine monophosphate (AMP), glutathione, water-soluble nucleotides, such as inosine monophosphate, disodium inosinate, xanthosine monophosphate, guanylate monophosphate, alapyridaine (N-(1-carboxyethyl)-6-(hydroxymethyl)pyridinium-3-ol inner salt, sugar beet extract (alcoholic extract), sugarcane leaf essence (alcoholic extract), curculin, strogin, mabinlin, gymnemic acid, 2-hydroxybenzoic acid (2-HB), 3-hydroxybenzoic acid (3-HB), 4-hydroxybenzoic acid (4-HB), 2,3-dihydroxybenzoic acid (2,3-DHB), 2,4-dihydroxybenzoic acid (2,4-DHB), 2,5-dihydroxybenzoic acid (2,5-DHB), 2,6-dihydroxybenzoic acid (2,6-DHB), 3,4-dihydroxybenzoic acid (3,4-DHB), 3,5-dihydroxybenzoic acid (3,5-DHB), 2,3,4-trihydroxybenzoic acid (2,3,4-THB), 2,4,6-trihydroxybenzoic acid (2,4,6-THB), 3,4,5-trihydroxybenzoic acid (3,4,5-THB), 4-hydroxyphenylacetic acid, 2-hydroxyisocaproic acid, 3-hydroxycinnamic acid, 3-aminobenzoic acid, 4-aminobenzoic acid, 4-methoxysalicylic acid, and combinations thereof.

Other suitable taste potentiators are substantially or completely insoluble in water, such as citrus aurantium, vanilla oleoresin, water insoluble sugar acids, water insoluble hydrolyzed vegetable proteins, water insoluble hydrolyzed animal proteins, water insoluble yeast extracts, insoluble nucleotides, sugarcane leaf essence. and combinations thereof. Some other suitable taste potentiators include substances that are slightly soluble in water, such as maltol, ethyl maltol, vanillin, slightly water-soluble sugar acids, slightly water-soluble hydrolyzed vegetable proteins, slightly water-soluble hydrolyzed animal proteins, slightly water-soluble yeast extracts, slightly water-soluble nucleotides and combinations thereof.

As mentioned above, sweetener potentiators, which are a type of taste potentiator, enhance the taste of sweetness. Exemplary sweetener potentiators include monoammonium glycyrrhizinate, licorice glycyrrhizinates, citrus aurantium, alapyridaine, alapyridaine (N-(1-carboxyethyl)-6-(hydroxymethyl)pyridinium-3-ol) inner salt, miraculin, curculin, strogin, mabinlin, gymnemic acid, cynarin, glupyridaine, pyridinium-betain compounds, sugar beet extract, neotame, thaumatin, neohesperidin dihydrochalcone, tagatose, trehalose, maltol, ethyl maltol, vanilla extract, vanilla oleoresin, vanillin, sugar beet extract (alcoholic extract), sugarcane leaf essence (alcoholic extract), compounds that respond to G-protein coupled receptors (T2Rs and T1Rs, 2-hydroxybenzoic acid (2-HB), 3-hydroxybenzoic acid (3-HB), 4-hydroxybenzoic acid (4-HB), 2,3-dihydroxybenzoic acid (2,3-DHB), 2,4-dihydroxybenzoic acid (2,4-DHB), 2,5-dihydroxybenzoic acid (2,5-DHB), 2,6-dihydroxybenzoic acid (2,6-DHB), 3,4-dihydroxybenzoic acid (3,4-DHB), 3,5-dihydroxybenzoic acid (3,5-DHB), 2,3,4-trihydroxybenzoic acid (2,3,4-THB), 2,4,6-trihydroxybenzoic acid (2,4,6-THB), 3,4,5-trihydroxybenzoic acid (3,4,5-THB), 4-hydroxyphenylacetic acid, 2-hydroxyisocaproic acid, 3-hydroxycinnamic acid, 3-aminobenzoic acid, 4-aminobenzoic acid, 4-methoxysalicylic acid and combinations thereof.

The sensation of warming or cooling effects can also be prolonged with the use of a hydrophobic sweetener as described in U.S. Patent Publication No. 2003/0072842 A1 of Johnson et al. For example, such hydrophobic sweeteners include those of the formulae I-XI as set forth below: 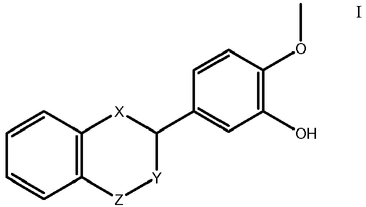 wherein X, Y and Z are independently at each occurrence selected from the group consisting of CH₂, O and S; 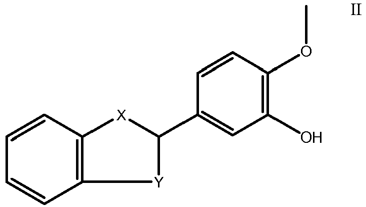 wherein X and Y are independently at each occurrence selected from the group consisting of S and O; 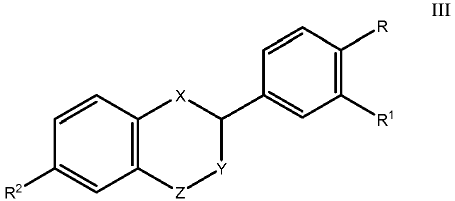 wherein X is S or O; Y is O or CH₂; Z is CH₂, SO₂ or S; R is OCH₃, OH or H; R¹ is SH or OH and R² is H or OH; 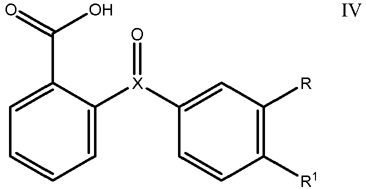 wherein X is C or S; R is OH or H and R¹ is OCH₃ or OH; 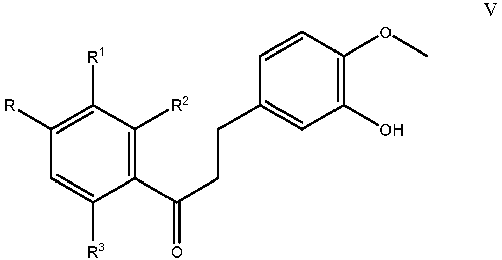 wherein R, R² and R³ are OH or H and R¹ is H or COOH; 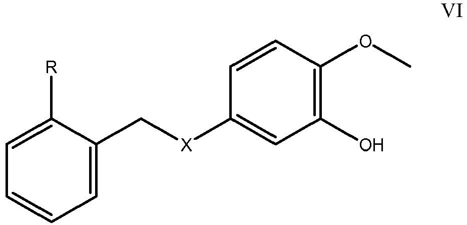 wherein X is O or CH₂ and R is COOH or H; 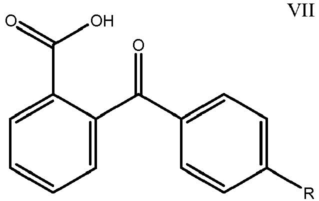 wherein R is CH₃CH₂, OH, N (CH₃)₂ or Cl; 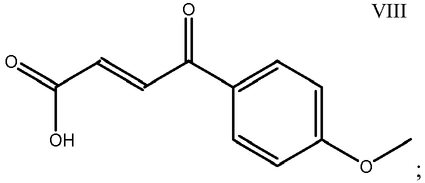 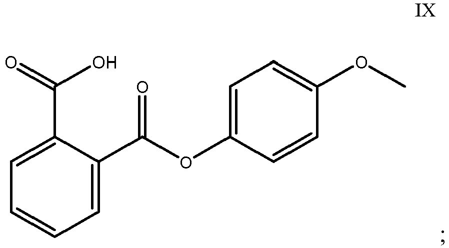 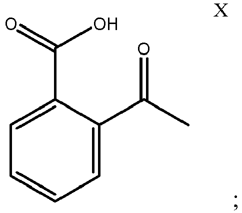 and

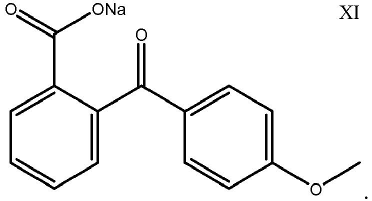

Perillartine also can be added as described in U.S. Patent No. 6,159,509 to Johnson et al.

Any of the above-listed taste potentiators can be used alone or in combination.

Some embodiments include two or more taste potentiators that act synergistically with one another. For instance, in some embodiments, a sweetener potentiator composition can be provided, which includes two or more sweetener potentiators that act synergistically with one another. The sweetener potentiator composition can enhance the sweetness of products into which it is incorporated by reducing the amount of sucrose needed to provide a sweetness intensity equivalent to sucrose. The sweetness enhancing effect of the combination of sweetener potentiators can be greater than the effect of either compound used individually.

Additional taste potentiators include those described, for example, in U.S. Patent Nos. 5,631,038 and 6,008,250 to Kurtz et al. In some embodiments, the taste potentiator can comprise 3-hydroxybenzoic acid and a dihydroxybenzoic acid selected from the group consisting of 2,4-dihydroxybenzoic acid, 3,4-dihydroxybenzoic acid, and combinations thereof. Comestible salts, such as sodium, potassium salts, calcium, magnesium, and ammonium salts, can be substituted for the free acids in these potentiator combinations.

The confectionery base typically contributes sweetness to the confection. Additional sweeteners, specifically high-intensity sweeteners, can also be used. High-intensity sweeteners include, for example, the potassium salt of 3,4-dihydro-6-methyl-1,2,3-oxathiazine-4-one-2,2-dioxide, L-aspartyl-L-phenylalanine methyl ester, L-alpha-aspartyl-N-(2,2,4,4-tetramethyl-3-thietanyl)-D-alaninamide hydrate, N-[N-(3,3-dimethylbutyl)-L-aspartyl]-L-phenylalanine 1-methyl ester, chlorinated derivatives of sucrose, thaumatin, monatin, mogrosides, rebaudiosides (including Rebaudioside A), and combinations thereof.

The confection can, optionally, further include a flavorant. Suitable flavorants include artificial or natural flavors known in the art, for example synthetic flavor oils, natural flavoring aromatics and/or oils, oleoresins, extracts derived from plants, leaves, flowers, fruits, and the like, and combinations thereof. Nonlimiting representative flavors include oils such as spearmint oil, cinnamon oil, oil of wintergreen (methyl salicylate), peppermint oil, clove oil, bay oil, anise oil, eucalyptus oil, thyme oil, cedar leaf oil, oil of nutmeg, allspice, oil of sage, mace, oil of bitter almonds, cassia oil, citrus oils including lemon, orange, lime, grapefruit, vanilla, fruit essences, including apple, pear, peach, grape, strawberry, raspberry, blackberry, cherry, plum, pineapple, apricot, banana, melon, tropical fruit, mango, mangosteen, pomegranate, papaya, and honey lemon essences, and the like, and combinations thereof. Other types of flavorants can include various aldehydes and esters such as cinnamyl acetate, cinnamaldehyde, citral diethylacetal, dihydrocarvyl acetate, eugenyl formate, p-methylamisol, acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2-hexenal (berry fruits), tolyl aldehyde (cherry, almond), veratraldehyde (vanilla), 2,6-dimethyl-5-heptenal, i.e., melonal (melon), 2,6-dimethyloctanal (green fruit), and 2-dodecenal (citrus, mandarin). Flavorants can be used in liquid or solid form. When used in solid (dry) form, suitable drying means such as spray drying the oil can be used.

In the confection, the physiological cooling agent(s) can, optionally, be combined with a warming agent. Warming agents include a wide variety of compounds known to provide the sensory signal of warming to the user. These compounds offer the perceived sensation of warmth, particularly in the oral cavity, and often enhance the perception of flavors, sweeteners and other organoleptic components. Suitable warming agents include vanillyl alcohol n-butylether (TK-1000) supplied by Takasago Perfumary Company Limited, Tokyo, Japan, vanillyl alcohol n-propylether, vanillyl alcohol isopropylether, vanillyl alcohol isobutylether, vanillyl alcohol n-aminoether, vanillyl alcohol isoamyl ether, vanillyl alcohol n-hexyl ether, vanillyl alcohol methyl ether, vanillyl alcohol ethyl ether, gingerol, shogaol, paradol, zingerone, capsaicin, dihydrocapsaicin, nordihydrocapsaicin, homocapsaicin, homodihydrocapsaicin, ethanol, isopropyl alcohol, isoamyl alcohol, benzyl alcohol, glycerine, and combinations thereof. In some embodiments, a warming agent and a cooling agent can be incorporated into spatially distinct regions of the confection.

The confection can, optionally, further include a breath freshener. Breath fresheners can include zinc citrate, zinc acetate, zinc fluoride, zinc ammonium sulfate, zinc bromide, zinc iodide, zinc chloride, zinc nitrate, zinc fluorosilicate, zinc gluconate, zinc tartarate, zinc succinate, zinc formate, zinc chromate, zinc phenol sulfonate, zinc dithionate, zinc sulfate, silver nitrate, zinc salicylate, zinc glycerophosphate, copper nitrate, chlorophyll, copper chlorophyll, chlorophyllin, hydrogenated cottonseed oil, chlorine dioxide, beta cyclodextrin, zeolite, silica-based material, carbon-based material, enzymes such as laccase, and combinations thereof. Breath fresheners can include essential oils as well as various aldehydes and alcohols. Essential oils used as breath fresheners can include oils of spearmint, peppermint, wintergreen, sassafras, chlorophyll, citral, geraniol, cardamom, clove, sage, carvacrol, eucalyptus, cardamom, magnolia bark extract, marjoram, cinnamon, lemon, lime, grapefruit, orange, and combinations thereof. Aldehydes such as cinnamic aldehyde and salicylaldehyde can be used. Additionally, chemicals such as carvone, iso-garrigol, and anethole can function as breath fresheners.

The confection can, optionally, further include a mouth moistener. Suitable mouth moisteners can include saliva stimulators such as acids and salts including acetic acid, adipic acid, ascorbic acid, butyric acid, citric acid, formic acid, fumaric acid, glyconic acid, lactic acid, phosphoric acid, malic acid, oxalic acid, succinic acid, and tartaric acid. Mouth moisteners can also include hydrocolloid materials that hydrate and can adhere to oral surface to provide a sensation of mouth moistening. Hydrocolloid materials can include naturally occurring materials such as plant exudates, seed gums, and seaweed extracts or they can be chemically modified materials such as cellulose, starch, or natural gum derivatives. Furthermore, hydrocolloid materials can include pectin, gum arabic, acacia gum, alginates, agar, carageenans, guar gum, xanthan gum, locust bean gum, gelatin, gellan gum, galactomannans, tragacanth gum, karaya gum, curdlan, konjac, chitosan, xyloglucan, beta glucan, furcellaran, gum ghatti, tamarin, and bacterial gums. Mouth moisteners can include modified natural gums such as propylene glycol alginate, carboxymethyl locust bean gum, low methoxyl pectin, and combinations thereof. Modified celluloses can be included such as microcrystalline cellulose, carboxymethylcellulose (CMC), methylcellulose (MC), hydroxypropylmethylcellulose (HPCM), hydroxypropylcellulose (HPC), and combinations thereof.

Coloring agents can be used to produce a desired color for the confection. Suitable coloring agents include pigments and natural food colors and dyes suitable for food, drug, and cosmetic applications. Suitable food colors include annatto extract (E160b), bixin, norbixin, astaxanthin, dehydrated beets (beet powder), beetroot red/betanin (E162), ultramarine blue, canthaxanthin (E161g), cryptoxanthin (E161c), rubixanthin (E161d), violanxanthin (E161e), rhodoxanthin (E161f), caramel (E150(a-d)), beta-apo-8-carotenal (E160e), carotene (E160a), alpha carotene, gamma carotene, ethyl ester of beta-apo-8-carotenal (E160f), flavoxanthin (E161a), lutein (E161b), cochineal extract (E120), carmine (E132), carmoisine/azorubine (E122), sodium copper chlorophyllin (E141), chlorophyll (E140), toasted partially defatted cooked cottonseed flour, ferrous gluconate, ferrous lactate, grape color extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminum (E173), silver (E174), gold (E175), pigment rubine/lithol rubine BK (E180), calcium carbonate (E170), carbon black (E153), black PN/brilliant black BN (E151), green S/acid brilliant green BS (E142), and combinations thereof. In some embodiments, certified colors can include FD&C aluminum lakes, and combinations thereof.

The confection can, optionally, further include an acidulant. Suitable acidulants include acetic, citric, fumaric, hydrochloric, lactic, and nitric acids as well as sodium citrate, sodium bicarbonate and carbonate, sodium or potassium phosphate and magnesium oxide, potassium metaphosphate, sodium acetate, and combinations thereof.

The confection can, optionally, further include a buffering agent. Exemplary buffering agents can include sodium bicarbonate, sodium phosphate, sodium hydroxide, ammonium hydroxide, potassium hydroxide, sodium stannate, triethanolamine, citric acid, hydrochloric acid, sodium citrate, and combinations thereof.

The confection can, optionally, further include an antioxidant. Antioxidants include butylated hydroxytoluene (BHT), butylated hydroxyanisole (BHA), propyl gallate, and combinations thereof.

The confection can, optionally, further include a nutraceutical. Suitable nutraceuticals include herbs and botanicals such as aloe, bilberry, bloodroot, calendula, capsicum, chamomile, cat's claw, echinacea, garlic, ginger, ginkgo, goldenseal, various ginseng, green tea, guarana, kava kava, lutein, nettle, passionflower, rosemary, saw palmetto, St. John's wort, thyme, and valerian. Also included are mineral supplements such as calcium, copper, iodine, iron, magnesium, manganese, molybdenum, phosphorous, zinc, and selenium. Other nutraceuticals can include fructooligosaccharides, glucosamine, grapeseed extract, cola extract, guarana, ephedra, inulin, phytosterols, phytochemicals, catechins, epicatechin, epicatechin gallate, epigallocatechin, epigallocatechin gallate, isoflavones, lecithin, lycopene, oligofructose, polyphenols, flavanoids, flavanols, flavonols, and psyllium as well as weight loss agents such as chromium picolinate and phenylpropanolamine. Exemplary vitamins and co-enzymes include water or fat soluble vitamins such as thiamin, riboflavin, nicotinic acid, pyridoxine, pantothenic acid, biotin, folic acid, flavin, choline, inositol and para-aminobenzoic acid, carnitine, vitamin C, vitamin D and its analogs, vitamin A and the carotenoids, retinoic acid, vitamin E, vitamin K, vitamin B₆, and vitamin B₁₂. Combinations of the foregoing nutraceuticals can be used.

The confection can, optionally, further include a medicament. Suitable medicaments can include oral care agents, throat care agents, allergy relief agents, and general medical care agents.

Suitable oral care agents include breath fresheners, tooth whiteners, antimicrobial agents, tooth mineralizers, tooth decay inhibitors, topical anesthetics, mucoprotectants, stain removers, oral cleaning, bleaching agents, desensitizing agents, dental remineralization agents, antibacterial agents, anticaries agents, plaque acid buffering agents, surfactants, and anticalculus agents, and combinations thereof. Examples of such ingredients include hydrolytic agents such as proteolytic enzymes, abrasives such as hydrated silica, calcium carbonate, sodium bicarbonate and alumina, other active stain-removing components such as surface-active agents, including anionic surfactants such as sodium stearate, sodium palmitate, sulfated butyl oleate, sodium oleate, salts of fumaric acid, glycerol, hydroxylated lecithin, sodium lauryl sulfate and chelators such as polyphosphates, which are typically employed as tartar control ingredients. Oral care agents also include tetrasodium pyrophosphate and sodium tri-polyphosphate, sodium bicarbonate, sodium acid pyrophosphate, sodium tripolyphosphate, xylitol, sodium hexametaphosphate, peroxides such as carbamide peroxide, calcium peroxide, magnesium peroxide, sodium peroxide, hydrogen peroxide, and peroxydiphospate.

In addition, oral care ingredients also include antibacterial agents comprising triclosan, chlorhexidine, zinc citrate, silver nitrate, copper, limonene, cetyl pyridinium chloride, and combinations thereof.

Anticaries agents include fluoride ions, fluorine-providing components (e.g., inorganic fluoride salts), soluble alkali metal salts (e.g., sodium fluoride, potassium fluoride, sodium fluorosilicate, ammonium fluorosilicate, potassium fluoride, sodium monofluorophosphate), and tin fluorides, (e.g., stannous fluoride and stannous chloride, potassium stannous fluoride (SnF₂-KF), sodium hexafluorostannate, stannous chlorofluoride), and combinations thereof.

Lubricants also can be added in some embodiments to improve the smoothness of the comestible, such as, for example hard candy embodiments. Smoothness also is a characteristic that leads to an increased perception of mouth-moistening upon consumption. Suitable lubricants include, for example, fats, oils, aloe vera, pectin, and the like, and combinations thereof.

Similarly, in some embodiments, the comestible can have smooth edges. In such embodiments, the comestible can have any shape, such as square, circular or diamond-shaped, however, the edges are rounded to provide a smooth comestible. Another manner of lending smoothness to the comestibles is to deposit the comestible composition into molds during the manufacturing process. Accordingly, in some embodiments, the comestible is deposited, as described in more detail below.

Hard candy confectionery compositions can be prepared using conventional methods and equipments, such as fire cookers, vacuum cookers, and scraped-surface cookers (also referred to as high speed atmospheric cookers). When using a fire cooker, the desired quantity of confectionery base is dissolved in water by heating the agent in a kettle until the confectionery base dissolves. Additional confectionery base can then be added and cooking continued until a final temperature of, for example, 145° to 156°C is achieved. The batch is then cooled and worked as a plastic-like mass to incorporate additives separately or in the form of one or more concentrates or pre-mixes.

Center filling compositions can be prepared by blending the component, optionally in the presence of heat, to form an intimate blend.

Methods of forming confections of uniform composition are known in the art and include forming a rope of the above-described plastic-like mass, and dividing the rope into individual confections of desire shape and weight.

Methods of forming center-filled confections are known in the art and include the co-deposition technique described in U.S. Patent No. 4,517,205 to Aldrich.

The invention is further illustrated by the following non-limiting examples. EXAMPLES 1 and 2

These experiments illustrate the surprising differences in regioselectivity of cooling exhibited by N-ethyl-2,2-diisopropylbutanamide and N,2,3 -trimethyl-2-isopropylbutanamide. These differences are particularly surprising given the small difference in the respective chemical structures, as noted above.

The cooling agents N-ethyl-2,2-diisopropylbutanamide and N,2,3-trimethyl-2-isopropylbutanamide were evaluated in fondant at concentrations of 0.03, 0.06, and 0.09 weight percent. The fondant base consisted of 85 weight percent 6X fondant sugar and 15 weight percent deionized water.

Sensory evaluations of the cooling agent-containing fondants were conducted by six evaluators, who observed the location, intensity, and onset speed of cooling sensation as they tasted approximately 0.5 gram of fondant by allowing it to dissolve in the mouth. Results are summarized in Table 1. The results show that, relative to N,2,3-trimethyl-2-isopropylbutanamide, N-ethyl-2,2-diisopropylbutanamide provides superior and unexpected throat cooling when used in a candy system.

**Table 1**

| | Example 1 (N-ethyl-2,2-diisopropylbutanamide) | Example 2 (N,2,3-trimethyl-2-isopropylbutanamide) |
|---|---|---|
| Predominant mouth cooling | No | Yes |
| Long-lasting mouth cooling | No | No |
| Predominant throat cooling | Yes | No |
| Long-lasting throat cooling | Yes | No |

### EXAMPLES 3-6

These examples illustrate formulations of center-filled confections for throat soothing. The confections consist of a hard candy shell and a fluid center filling.

Representative compositions for two hard candy shell compositions are provided in Table 2, where all component amounts are expressed in weight percent based on the total hard candy shell composition.

Hard candy shell compositions are prepared by combining sweeteners (e.g., bulk sweetener) as well as a solvent (e.g., water), in a mixing vessel for about four to about ten minutes to form a slurry. The slurry is heated to about 70°C to 120°C to dissolve any sweetener crystals or particles and form an aqueous solution. Once dissolved, heat at temperatures of about 135°C to 160°C and vacuum are applied to cook the batch and boil off water until a residual moisture of less than about 4% is achieved. The batch changes from a crystalline to an amorphous phase. The flavor agent, food-grade acid composition and optionally cooling agent(s) are then admixed in the batch by mechanical mixing operations, along with any other optional additives, such as coloring agents.

The batch is then cooled to about 100°C to 20°C to attain a semi-solid or plastic-like consistency. Once the candy mass has been properly tempered, it ready for forming into desired hard candy shell shapes having the correct weight and dimensions.

The sucrose/glucose mixture illustrated by Examples 3 and 4 can be partially or completely replaced by hydrogenated isomaltulose, maltitol, hydrogenated starch hydrolysate, and mixtures thereof.

**Table 2. Hard Candy Shell Compositions**

| Component | Example 3 | Example 4 |
|---|---|---|
| Sucrose | 45 - 65 | 45 - 65 |
| Glucose | 40 - 55 | 40 - 55 |
| Lemon Flavor | 0.25 | 0 |
| Strawberry Flavor | 0 | 0.325 |
| Menthol | 0.089 | 0.089 |
| Eucalyptus oil | 0.0066 | 0.0066 |
| Monomenthyl glutarate | 0.125 | 0.125 |
| N-Ethyl-2,2-diisopropylbutanamide | 0.09 | 0.09 |
| Lubricants | 0.9998 | 0.9998 |
| 2 weight % aqueous solution of beta-carotene | 0.15 | 0 |
| FD&C Red 40 | 0 | 0.0019 |
| 50 weight % aqueous solution of citric acid | 1.0 | 0.36 |
| 50 weight % aqueous solution of potassium citrate monohydrate | 0.336 | 0.12 |
| Water | 0.5-5 | 0.5-5 |

Representative compositions for two fluid center filling compositions are provided in Table 3, where all component amounts are expressed in weight percent based on the total fluid center filling composition. The fluid center filling compositions are prepared by blending all the components to form an intimate blend.

**Table 3. Fluid Center Filling Compositions**

| Component | Example 5 | Example 6 |
|---|---|---|
| High fructose glucose syrup | 80-95 | 80-95 |
| Glycerin | 2-20 | 2-20 |
| Sucrose | 2-10 | 2-10 |
| Lemon flavor | 0.192 | 0 |
| Strawberry flavor | 0 | 0.25 |
| Menthol | 0.06 | 0.045 |
| Monomenthyl glutarate | 0.125 | 0.125 |
| N-Ethyl-2,2-diisopropylbutanamide | 0.09 | 0.09 |

The finished center-filled confections consist of about 83 to about 93 weight percent of a hard candy shell according to one of the compositions of Table 2, and about 7 to about 17 weight percent of a fluid center filling according to one of the compositions of Table 3.

### EXAMPLES 7-21

These examples illustrate various center-fill compositions with fat carriers (Table 4; Examples 7-10), shell compositions (Table 5; Examples 11-17), and coating compositions (Table 6; Examples 18-21). Collectively, these examples can be used to form multi-region confectioner compositions. In Tables 4-6, component amounts are expressed in weight percent based on the total weight of the composition.

Multi-region chewy confectionery compositions including a filling region, a shell region, and optionally a coating region are prepared according to the compositions in Tables 4-6 with each region according to the corresponding components for Examples 5-19.

To prepare the filling compositions, the powdered ingredients are combined in a mixing vessel and mixed until homogeneous. Next, the fat and emulsifier are melted together. The N-ethyl-2,2-diisopropylbutanamide and any other cooling compounds are solubilized in the flavor and added along with the acid to the melted fat/emulsifier blend. Once the fat mixture is homogeneously mixed, the powders are mixed in until homogeneously dispersed.

To prepare the shell region, the sucrose with corn syrups and water or polyol (maltitol) and water are mixed to create a homogeneous mixture and the mixture is cooked to about 130°C. Separately, the gelatin is hydrated by mixing with a small amount of water. When the cooked mixture reaches about 90°C, the hydrated gelatin is added along with fat, emulsifier, color, high intensity sweetener(s) and acid and mixed thoroughly. The mixture is then placed on a cooling table where flavor (including N-ethyl-2,2-diisopropylbutanamide and any other cooling compounds solubilized in the flavor) and acid are added. The mixture is then pulled to a desired consistency before being fed into a process for forming a multi-region chewy confectionery product.

The shell and filling compositions are then extruded together and formed into any desired shape configuration. An optional coating composition as shown in Table 6 can be applied as described above in the section describing the coating region. The confectionery pieces can each have a total weight of approximately 2 to 10 grams. In the final confectionery pieces, the confectionery region is about 40-65% by weight, the filling is about 5-45% by weight, and the coating is about 0-50% by weight.

**Table 4. Filling Compositions with Fat Carriers**

| | Ex. 7 | Ex. 8 | Ex. 9 | Ex. 10 |
|---|---|---|---|---|
| Dextrose powder | 20-80 | 0 | 10-40 | 0 |
| Erythritol powder | 0 | 20-80 | 10-40 | 10-40 |
| Xylitol powder | 0 | 0 | 0 | 10-40 |
| Hydrogenated vegetable fat | 20-80 | 20-80 | 20-80 | 0 |
| Cocoa fat | 0 | 0 | 0 | 20-80 |
| Emulsifier | 0-5 | 0-5 | 0-5 | 0-5 |
| Food acid | 0-5 | 0-5 | 0-5 | 0-5 |
| Flavor | 0-5 | 0-5 | 0-5 | 0-5 |
| N-Ethyl-2,2-diisopropylbutanamide | 0.02-0.5 | 0.02-0.5 | 0.02-0.5 | 0.02-0.5 |
| Color | 0-0.5 | 0-0.5 | 0-0.5 | 0-0.5 |

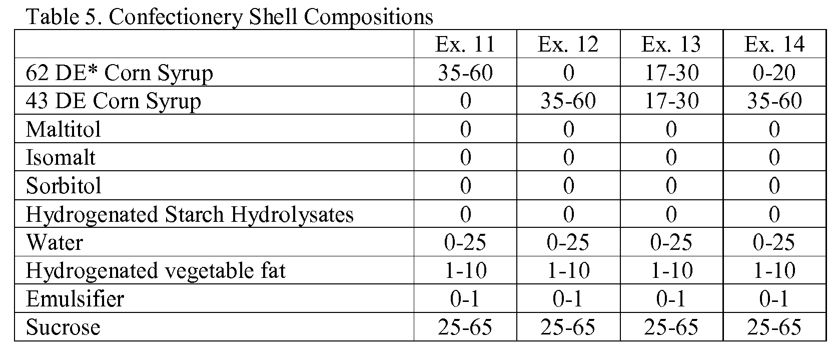

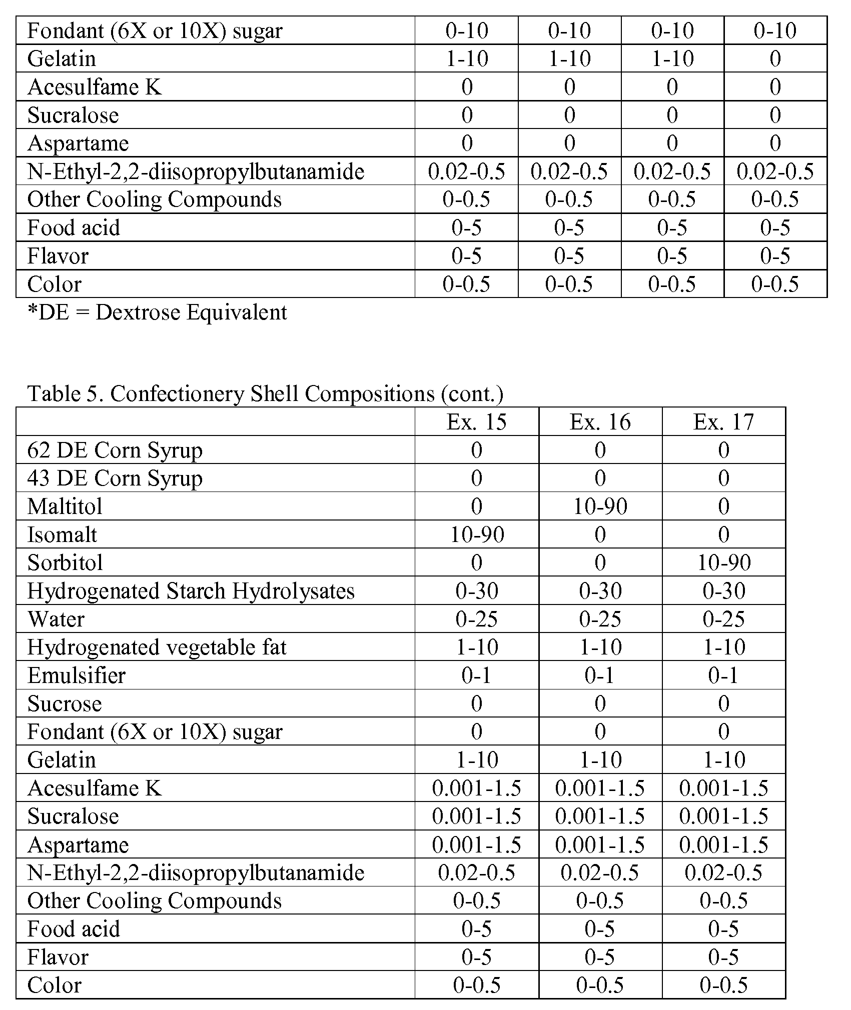

**Table 6. Confectionery Coating Compositions**

| | Ex. 18 | Ex. 19 | Ex. 20 | Ex. 21 |
|---|---|---|---|---|
| Sucrose | 90-99.9 | 0 | 0 | 0 |
| Dextrose | 0 | 90-99.9 | 0 | 0 |
| Maltitol | 0 | 0 | 0 | 90-99.9 |
| Isomalt | 0 | 0 | 90-99.9 | 0 |
| Gum Arabic | 0-5 | 0-5 | 0-5 | 0-5 |
| Starch | 0-5 | 0-5 | 0-5 | 0-5 |
| N-Ethyl-2,2-diisopropylbutanamide | 0.02-0.5 | 0.02-0.5 | 0.02-0.5 | 0.02-0.5 |
| Other Cooling Compounds | 0-0.5 | 0-0.5 | 0-0.5 | 0-0.5 |
| Flavor | 0-5 | 0-5 | 0-5 | 0-5 |
| Color | 0-0.5 | 0-0.5 | 0-0.5 | 0-0.5 |

### EXAMPLES 22-25

These examples illustrate powder filling compositions comprising N-ethyl-2,2-diisopropylbutanamide. Powder filling compositions are summarized in Table 7, where all amounts are in parts by weight based on the total weight of the powder filling compositions. To prepare the filling compositions, the powdered ingredients are combined in a mixing vessel and mixed until homogeneous. The powder filling compositions can be combined with the confectionery shell compositions of Table 5, and, optionally, the confectionery coating compositions of Table 6 to form center-filled confections. In the final confectionery pieces, the shell region is about 40-65% by weight, the filling is about 5-45% by weight, and the coating is about 0-50% by weight.

**Table 7. Filling compositions with Particulate Materials**

| | Ex. 22 | Ex. 23 | Ex. 24 | Ex. 25 |
|---|---|---|---|---|
| Dextrose powder | 90-100 | 0 | 45-50 | 0 |
| Erythritol powder | 0 | 90-100 | 45-50 | 45-50 |
| Xylitol powder | 0 | 0 | 0 | 45-50 |
| Food acid | 0-5 | 0-5 | 0-5 | 0-5 |
| Flavor | 0-5 | 0-5 | 0-5 | 0-5 |
| N-Ethyl-2,2-diisopropylbutanamide | 0.02-0.5 | 0.02-0.5 | 0.02-0.5 | 0.02-0.5 |
| Color | 0-0.5 | 0-0.5 | 0-0.5 | 0-0.5 |
| Bulk density | 0.5-0.6 | 0.2-0.3 | 0.3-0.4 | 0.3-0.4 |
| Tap density | 0.6-0.7 | 0.5-0.6 | 0.6-0.7 | 0.6-0.7 |

### EXAMPLES 26-29

These examples illustrate center-fill compositions comprising N-ethyl-2,2-diisopropylbutanamide and aqueous carriers. The center-fill compositions are summarized in Table 8, where all amounts are in parts by weight based on the total weight of the powder filling compositions. To prepare the center-fill compositions, the hydrogenated starch hydrolysates and/or corn syrup(s) are mixed together with any glycerin until homogeneous. Then the powdered ingredients, flavor, acid, color, cooling agent, and emulsifiers are combined into the liquid blend and mixed until homogeneous. The powder filling compositions can be combined with the confectionery shell compositions of Table 5, and, optionally, the confectionery coating compositions of Table 6 to form center-filled confections. In the final confectionery pieces, the shell region is about 40-65% by weight, the filling is about 5-45% by weight, and the coating is about 0-50% by weight.

**Table 8. Filling compositions with Aqueous Carriers**

| | Ex. 26 | Ex. 27 | Ex. 28 | Ex. 29 |
|---|---|---|---|---|
| Dextrose powder | 15-35 | 0 | 15-35 | 0 |
| Erythritol powder | 15-35 | 35-65 | 15-35 | 15-35 |
| Xylitol powder | 0 | 0 | 0 | 15-35 |
| Hydrogenated Starch Hydrolysate | 0 | 0 | 15-75 | 15-75 |
| Glycerin | 0-20 | 0-20 | 0-20 | 0-20 |
| High Fructose Corn Syrup | 5-15 | 5-15 | 0 | 0 |
| High Maltose Corn Syrup | 0-10 | 0-10 | 0 | 0 |
| Invert Sugar | 10-30 | 10-30 | 0 | 0 |
| Carboxymethyl Cellulose | 0-0.2 | 0-0.2 | 0 | 0 |
| Emulsifier | 0-5 | 0-5 | 0-5 | 0-5 |
| Food acid | 0-5 | 0-5 | 0-5 | 0-5 |
| Flavor | 0-5 | 0-5 | 0-5 | 0-5 |
| N-Ethyl-2,2-diisopropylbutanamide | 0.02-0.5 | 0.02-0.5 | 0.02-0.5 | 0.02-0.5 |
| Color | 0-0.5 | 0-0.5 | 0-0.5 | 0-0.5 |

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

All ranges disclosed herein are inclusive of the endpoints, and the endpoints are independently combinable with each other.

The use of the terms "a" and "an" and "the" and similar referents in the context of describing the invention (especially in the context of the following claims) are to be construed to cover both the singular and the plural, unless otherwise indicated herein or clearly contradicted by context. Further, it should further be noted that the terms "first," "second," and the like herein do not denote any order, quantity, or importance, but rather are used to distinguish one element from another. The modifier "about" used in connection with a quantity is inclusive of the stated value and has the meaning dictated by the context (e.g., it includes the degree of error associated with measurement of the particular quantity).

## Claims

1. A confection, comprising:
at least 80 weight percent of a confectionery base;
0.005 to 0.5 weight percent of monomenthyl glutarate; and
0.03 to 0.4 weight percent of N-ethyl-2,2-diisopropylbutanamide;
wherein all weight percents are based on the total weight of the confection.

2. The confection of claim 1, wherein the confection is a hard candy confection.

3. The confection of claim 1, wherein the confection is a soft candy confection.

4. The confection of any of claims 1-3, further comprising 0.01 to 0.2 weight percent menthol.

5. The confection of any of claims 1-4, further comprising 0.001 to 0.02 weight percent eucalyptus oil.

6. The confection of any of claims 1-5, wherein the confection is a center-filled confection comprising a shell and a center filling.

7. The confection of claim 6,
wherein the shell comprises a hard candy shell composition comprising, based on the total weight of the confection composition,
at least 80 weight percent of a confectionery base;
0.005 to 0.5 weight percent of monomenthyl glutarate; and
0.03 to 0.4 weight percent ofN-ethyl-2,2-diisopropylbutanamide; and
wherein the center filling comprises a powder center filling composition comprising, based on the total weight of the powder center filling composition,
90 to 99.99 weight percent of a powdered confectionery base;
0.5 to 25 weight percent of a powder flow agent selected from the group consisting of powdered cellulose, magnesium stearate, stearic acid, paraffin and microcrystalline waxes, polyethylene waxes, mineral and other lubricating oils, talc, silicone dioxide, lactose, calcium citrate, and mixtures thereof;
0.005 to 0.5 weight percent of monomenthyl glutarate; and
0.03 to 0.4 weight percent ofN-ethyl-2,2-diisopropylbutanamide,

8. The confection of claim 6,
wherein the shell comprises a hard candy shell composition comprising, based on the total weight of the confection composition,
at least 80 weight percent of a confectionery base;
0.005 to 0.5 weight percent of monomenthyl glutarate; and
0.03 to 0.4 weight percent ofN-ethyl-2,2-diisopropylbutanamide; and
wherein the center filling comprises a fluid center filling composition comprising, based on the total weight of the fluid center filling composition,
70 to 95 weight percent of a confectionery base;
5 to 20 weight percent of a viscosity modifier selected from the group consisting of glycerin, lecithin, medium chain triglycerides, and mixtures thereof;
0.005 to 0.5 weight percent of monomenthyl glutarate; and
0.03 to 0.4 weight percent ofN-ethyl-2,2-diisopropylbutanamide.

9. The confection of claim 6, wherein the shell, the center filling, or both comprise monomethyl glutarate.

10. The confection of claim 8,
wherein the center-filled confection comprises, based on the total weight of the center-filled confection, 70 to 95 weight percent of the hard candy shell composition and 5 to 30 weight percent of the fluid center filling composition;
wherein the hard candy shell composition comprises, based on the total weight of the hard candy shell composition,
90 to 99.5 weight percent of the confectionery base;
0.08 to 0.16 weight percent monomenthyl glutarate;
0.06 to 0.12 weight percent N-ethyl-2,2-diisopropylbutanamide; and
0.05 to 0.13 weight percent menthol;
wherein the fluid center filling comprises a fluid center filling composition comprising, based on the total weight of the fluid center filling,
75 to 90 weight percent confectionery base;
10 to 17 weight percent glycerin;
0.08 to 0.16 weight percent monomenthyl glutarate;
0.06 to 0.12 weight percent N-ethyl-2,2-diisopropylbutanamide; and 0.02 to 0.07 weight percent menthol.

11. Use of an effective amount ofN-ethyl-2,2-diisopropylbutanamide in the preparation of a confection for treatment of throat discomfort, wherein the confection has a mass of 2 to 10 grams and comprises 0.03 to 0.4 weight percent of N-ethyl-2,2-diisopropylbutanamide, based on the weight of the confection, and 0.005 to 0.5 weight percent of monomenthyl gluturate.

12. The use of claim 11, wherein the confection further comprises 0.01 to 0.2 weight percent menthol.

13. The use of any of claims 11-12, wherein the confection further comprises 0.001 to 0.02 weight percent eucalyptus oil.

## Patentansprüche

1. Süßware, enthaltend:
zumindest 80 Gew.-% einer Süßwarengrundmasse;
0,005 bis 0,5 Gew.-% Monomenthylglutarat; und
0,03 bis 0,4 Gew.-% N-ethyl-2,2-diisopropylbutanamid;
wobei alle Gew.-%-Angaben bezogen sind auf das Gesamtgewicht der Süßware.

2. Die Süßware nach Anspruch 1, wobei die Süßware eine Hartkaramelle ist.

3. Die Süßware nach Anspruch 1, wobei die Süßware eine Weichkaramelle ist.

4. Die Süßware nach einem der Ansprüche 1 bis 3, weiter enthaltend 0,01 bis 0,2 Gew.-% Menthol.

5. Die Süßware nach einem der Ansprüche 1 bis 4, weiter enthaltend 0,001 bis 0,02 Gew.-% Eukalyptusöl.

6. Die Süßware nach einem der Ansprüche 1 bis 5, wobei die Süßware eine gefüllte Süßigkeit, enthaltend eine Hülle und eine Kernfüllung, ist.

7. Die Süßware nach Anspruch 6,
wobei die Hülle eine Hartkaramellen-Hüllenzusammensetzung aufweist, welche,
bezogen auf das Gesamtgewicht der Süßwarenzusammensetzung,
zumindest 80 Gew.-% einer Süßwarengrundmasse;
0,005 bis 0,5 Gew.-% Monomenthylglutarat; und
0,03 bis 0,4 Gew.-% N-ethyl-2,2-diisopropylbutanamid enthält; und
wobei die Kernfüllung eine pulvrige Kernfüllungszusammensetzung aufweist, welche, bezogen auf das Gesamtgewicht der pulvrigen Kernfüllungszusammensetzung,
90 bis 99,99 Gew.-% einer pulvrigen Süßwarengrundmasse;
0,5 bis 25 Gew.-% eines Fließhilfsmittels, ausgewählt aus der Gruppe bestehend aus pulvriger Zellulose, Magnesiumstearat, Stearinsäure, Paraffin und mikrokristallinen Wachsen, Polyethylenwachsen, Mineralöl und anderen Schmierölen, Talkum, Siliziumdioxid, Laktose, Kalziumcitrat, und Mischungen davon;
0,005 bis 0,5 Gew.-% Monomenthylglutarat; und
0,03 bis 0,4 Gew.-% N-ethyl-2,2-diisopropylbutanamid, enthält.

8. Die Süßware nach Anspruch 6,
wobei die Hülle eine Hartkaramellen-Hüllenzusammensetzung aufweist, welche, bezogen auf das Gesamtgewicht der Süßwarenzusammensetzung,
zumindest 80 Gew.-% einer Süßwarengrundmasse;
0,005 bis 0,5 Gew.-% Monomenthylglutarat; und
0,03 bis 0,4 Gew.-% N-ethyl-2,2-diisopropylbutanamid enthält; und
wobei die Kernfüllung eine flüssige Kernfüllungszusammensetzung aufweist, welche, bezogen auf das Gesamtgewicht der flüssigen Kernfüllungszusammensetzung,
70 bis 95 Gew.-% einer Süßwarengrundmasse;
5 bis 20 Gew.-% eines Viskositätsmodifikators, ausgewählt aus der Gruppe bestehend aus Glycerin, Lecithin, mittelkettigen Triglyceriden, und Mischungen davon;
0,005 bis 0,5 Gew.-% Monomenthylglutarat; und
0,03 bis 0,4 Gew.-% N-ethyl-2,2-diisopropylbutanamid, enthält.

9. Die Süßware nach Anspruch 6, wobei die Hülle, die Kernfüllung oder beide Monomenthylglutarat enthalten.

10. Die Süßware nach Anspruch 8,
wobei die gefüllte Süßigkeit, bezogen auf das Gesamtgewicht der gefüllten Süßigkeit, 70 bis 95 Gew.-% der Hartkaramellen-Hüllenzusammensetzung und 5 bis 30 Gew.-% der flüssigen Kernfüllungszusammensetzung enthält;
wobei die Hartkaramellen-Hüllenzusammensetzung, bezogen auf das Gesamtgewicht der Hartkaramellen-Hüllenzusammensetzung,
90 bis 99,5 Gew.-% der Süßwarengrundmasse;
0,08 bis 0,16 Gew.-% Monomenthylglutarat;
0,06 bis 0,12 Gew.-% N-ethyl-2,2-diisopropylbutanamid; und
0,05 bis 0,13 Gew.-% Menthol enthält;
wobei die flüssige Kernfüllung eine flüssige Kernfüllungszusammensetzung aufweist, welche, bezogen auf das Gesamtgewicht der flüssigen Kernfüllung,
75 bis 90 Gew.-% Süßwarengrundmasse;
10 bis 17 Gew.-% Glycerin;
0,08 bis 0,16 Gew.-% Monomenthylglutarat;
0,06 bis 0,12 Gew.-% N-ethyl-2,2-diisopropylbutanamid; und
0,02 bis 0,07 Gew.-% Menthol enthält.

11. Verwendung einer wirksamen Menge an N-ethyl-2,2-diisopropylbutanamid zur Herstellung einer Süßware zur Behandlung von Halsschmerzen, wobei die Süßware ein Gewicht von 2 bis 10 g aufweist und 0,03 bis 0,4 Gew.-% N-ethyl-2,2-diisopropylbutanamid, bezogen auf das Gewicht der Süßware, und 0,005 bis 0,5 Gew.-% Monomenthylglutarat enthält.

12. Verwendung nach Anspruch 11, wobei die Süßware weiter 0,01 bis 0,2 Gew.-% Menthol enthält.

13. Verwendung nach Anspruch 11 bis 12, wobei die Süßware weiter 0,001 bis 0,02 Gew.-% Eukalyptusöl enthält.

## Revendications

1. Confection comprenant :
au moins 80 pourcent en poids d'une base de confiserie ;
de 0,005 à 0,5 pourcent en poids de glutarate de monomenthyle; et
de 0,03 à 0,4 pourcent en poids de N-éthyl-2,2-diisopropylbutanamide ;
tous les pourcentages en poids étant basés sur le poids total de la confection.

2. Confection suivant la revendication 1, dans laquelle la confection est une confection dure de bonbon.

3. Confection suivant la revendication 1, dans laquelle la confection est une confection molle de bonbon.

4. Confection suivant l'une quelconque des revendications 1 à 3, comprenant en outre de 0,01 à 0,2 pourcent en poids de menthol.

5. Confection suivant l'une quelconque des revendications 1 à 4, comprenant en outre de 0,001 à 0,02 pourcent en poids d'essence d'eucalyptus.

6. Confection suivant l'une quelconque des revendications 1 à 5, dans laquelle la confection est une confection à garnissage central, comprenant une coquille et un garnissage central.

7. Confection suivant la revendication 6,
dans laquelle la coquille comprend une composition de coquille de bonbon dure, comprenant, sur la base du poids total de la composition de confection,
au moins 80 pourcent en poids d'une base de confiserie ;
de 0,005 à 0,5 pourcent en poids du glutarate de monomenthyle ; et
de 0,03 à 0,4 pourcent en poids de N-éthyl-2,2-diisopropylbutanamide ; et
dans laquelle le garnissage central comprend une composition de garnissage central en poudre, comprenant, sur la base du poids total de la composition de garnissage central en poudre,
de 90 à 99,99 pourcent en poids une base de confiserie en poudre ;
de 0,5 à 25 pourcent en poids d'un agent de fluidification en poudre choisi dans le groupe, consistant en de la cellulose en poudre, du stéarate de magnésium, de l'acide stéarique, de la paraffine et des cires microcristallines, des cires de polyéthylène, des huiles minérales et autres huiles lubrifiantes, du talc, du dioxyde de silicium, du lactose, du citrate de calcium et leurs mélanges ;
de 0,005 à 0,5 pourcent en poids de glutarate de monomenthyle ; et
de 0,03 à 0,4 pourcent en poids de N-éthyl-2,2-diisopropylbutanamide.

8. Confection suivant la revendication 6, dans laquelle
la coquille comprend une composition de coquille de bonbon dure, comprenant, sur la base du poids total de la composition de la confection,
au moins 80 pourcent en poids d'une base de confiserie ;
de 0,005 à 0,5 pourcent en poids de glutarate de monomenthyle ; et
de 0,03 à 0,4 pourcent en poids de N-éthyl-2,2-diisopropylbutanamide ; et
dans laquelle le garnissage central comprend une composition fluide de garnissage central, comprenant, sur la base du poids total de la composition fluide de garnissage central,
de 70 à 95 pourcent en poids d'une base de confiserie ;
de 5 à 20 pourcent en poids d'un agent modifiant la viscosité, choisi dans le groupe consistant en la glycérine, une lécithine, des triglycérides à chaîne moyenne et leurs mélanges ;
de 0,005 à 0,5 pourcent en poids de glutarate de monomenthyle ; et
de 0,03 à 0,4 pourcent en poids de N-éthyl-2,2-diisopropylbutanamide.

9. Confection suivant la revendication 6, dans laquelle la coquille, le garnissage central ou les deux comprennent du glutarate de monomenthyle.

10. Confection suivant la revendication 8,
dans laquelle la confection garnie au centre comprend, sur la base du poids total de la confection garnie au centre, de 70 à 95 pourcent en poids de la composition de coquille de bonbon dure et de 5 à 30 pourcent en poids de la composition fluide de garnissage central ;
dans laquelle la composition de coquille de bonbon dure est de 5 à 30 pourcent en poids de la composition fluide de garnissage central ;
dans laquelle la composition de coquille de bonbon dure comprend sur la base du poids total de la composition dure de coquille de bonbon ;
de 90 à 99,5 pourcent en poids de la base de confiserie ;
de 0,08 à 0,16 en poids de glutarate de monomenthyle ;
de 0,06 à 0,12 pourcent en poids de N-éthyl-2,2-diisopropylbutanamide ; et
de 0,05 à 0,13 pourcent en poids de menthol ;
dans laquelle le garnissage central fluide comprend une composition de garnissage central fluide, comprenant, sur la base du poids total du garnissage central fluide ;
de 75 à 90 pourcent en poids d'une base de confiserie ;
de 10 à 17 pourcent en poids de glycérine ;
de 0,08 à 0,16 pourcent en poids de glutarate de monomenthyle ;
de 0,06 à 0,12 pourcent en poids de N-éthyl-2,2-diisopropylbutanamide ; et
de 0,02 à 0,07 pourcent en poids de menthol.

11. Utilisation d'une quantité efficace de N-éthyl-2,2-diisopropylbutanamide dans la préparation d'une confection de traitement d'un inconfort de la gorge, dans laquelle la confection a une masse de 2 à 10 grammes et comprend de 0,03 à 0,4 pourcent en poids de N-éthyl-2,2-diisopropylbutanamide sur la base du poids de la confection et de 0,005 à 0,5 pourcent en poids de glutarate de monomenthyle.

12. Utilisation suivant la revendication 11, dans laquelle la confection comprend en outre de 0,01 à 0,2 pourcent en poids de menthol.

13. Utilisation suivant l'une quelconque des revendications 11 à 12, dans laquelle la confection comprend en outre de 0,001 à 0,02 pourcent en poids d'essence d'eucalyptus.
